**Europäisches Patentamt**

**European Patent Office**

**Office européen des brevets**

(19)

(11) Veröffentlichungsnummer: **0 149 691**

**A1**

(12)

# EUROPÄISCHE PATENTANMELDUNG

(21) Anmeldenummer: **84100519.2**

(22) Anmeldetag: **19.01.84**

(51) Int. Cl.⁴: **F 04 B 43/10**
F 04 B 43/12, A 61 M 1/10

(43) Veröffentlichungstag der Anmeldung:
**31.07.85 Patentblatt 85/31**

(84) Benannte Vertragsstaaten:
**AT BE CH DE FR GB IT LI LU NL SE**

(71) Anmelder: **INTERMEDICAT GMBH**
**Gerliswilstrasse 74**
**CH-6020 Emmenbrücke(CH)**

(72) Erfinder: **Hessberg, Siegfried, Dipl.-Ing.**
**Im Nick 29**
**D-3508 Melsungen(DE)**

(72) Erfinder: **Dörfler, Josef**
**Talblick 2**
**D-3508 Melsungen 5(DE)**

(74) Vertreter: **Selting, Günther, Dipl.-Ing. et al,**
**Deichmannhaus am Hauptbahnhof**
**D-5000 Köln 1(DE)**

(54) **Schlauchpumpe, insbesondere für medizinische Anwendungen.**

(57) Die Schlauchpumpe weist ein Gehäuse (10) auf, in dem eine Antriebswelle (14) gelagert ist. Die Welle (14) ist mit zwei schräg verlaufenden Wellenstücken (20) versehen, auf welchem je eine Taumelscheibe gelagert ist. Jede Taumelscheibe, drückt mit ihrer Stirnseite gegen einen Schlauch (26), der in einer ringförmigen Nut eines Deckels (22) liegt. Jede Taumelscheibe rotiert relativ zu dem Gehäuse (10) und zu dem Schlauch (26) nicht und übt keine Schubkräfte auf den Schlauch (26) aus.

FIG.4

EP 0 149 691 A1

Croydon Printing Company Ltd.

PATENTANWÄLTE

Dr.-Ing. von Kreisler † 1973
Dr.-Ing. K. W. Eishold † 1981

Dr.-Ing. K. Schönwald
Dr. J. F. Fues
Dipl.-Chem. Alek von Kreisler
Dipl.-Chem. Carola Keller
Dipl.-Ing. G. Selting
Dr. H.-K. Werner

Intermedicat GmbH
Gerliswilstraße 74

CH-6020 Emmenbrücke

DEICHMANNHAUS AM HAUPTBAHNHOF

D-5000 KÖLN 1
Sg-Fe
18. Januar 1984

Schlauchpumpe, insbesondere für medizinische
Anwendungen

Die Erfindung betrifft eine Schlauchpumpe, insbesondere
für medizinische Anwendungen, mit einem Gehäuse, in dem
eine Antriebswelle gelagert ist, einem von der Antriebswelle schräg abstehenden Wellenstück, einer auf
dem Wellenstück gelagerten und gegenüber dem Gehäuse im
wesentlichen verdrehungssicher festgehaltenen Taumelscheibe, einem an der Stirnseite der Taumelscheibe anliegenden ringförmigen Schlauch, dessen Enden einen
Einlaß und einen Auslaß bilden, und mit einem an dem
Gehäuse angebrachten, den Schlauch abstützenden Deckel.

Bei einer zum Stand der Technik gehörenden, nicht vorveröffentlichen, Schlauchpumpe dieser Art (EP-Patentanmeldung 83 105 573.6) sind zwei Schläuche vorhanden,
die nebeneinander in einem gemeinsamen Gehäuse angeordnet sind. Jedem Schlauch ist eine eigene Taumelscheibe zugeordnet. Beide Schläuche haben einen gemeinsamen Zulauf und einen gemeinsamen Ablauf und ihre

0149691

Taumelkörper sind phasenmäßig versetzt oder gegenphasig angetrieben, um eine gleichmäßige Förderrate zu erzielen. Bei einer Schlauchpumpe setzt die Förderung kurzzeitig aus, wenn der den Schlauch zusammenquetschende Bereich der Taumelscheibe vom Auslaßende zum Einlaßende des Schlauchs überwechselt. Bei der genannten Schlauchpumpe tritt dieses Überwechseln zu unterschiedlichen Zeitpunkten auf, so daß sich die dadurch hervorgerufenen zeitlichen Änderungen der Fördermenge nicht auswirken. Mit der genannten Doppel-Schlauchpumpe kann wegen des einzigen Zulaufs nur eine einzige Flüssigkeit einem Patienten verabreicht werden.

Es gibt Fälle, in denen Pharmaka, kurz bevor sie dem Patienten verabreicht werden, miteinander gemischt werden müssen. Zur Durchführung einer solchen Mischung ist die vorgenannte Schlauchpumpe nicht imstande.

Der Erfindung liegt die Aufgabe zugrunde, eine Schlauchpumpe der eingangs genannten Art zu schaffen, mit der es möglich ist, zwei verschiedene Substanzen miteinander zu vermischen, unmittelbar bevor sie dem Patienten zugeführt werden.

Die Lösung dieser Aufgabe besteht erfindungsgemäß darin, daß zwei Schläuche seitlich nebeneinander in einer gemeinsamen Ebene angeordnet sind, daß jedem Schlauch eine eigene Taumelscheibe zugeordnet ist, daß die Taumelscheiben von einem gemeinsamen Antrieb mit gleichen Geschwindigkeiten angetrieben sind und daß beide Schläuche separate Zuläufe und einen gemeinsamen Ablauf aufweisen.

Mit der erfindungsgemäßen Schlauchpumpe können empfindliche Pharmaka, unmittelbar bevor sie dem Patienten verabreicht werden, miteinander gemischt werden. Die kurze Überleitungszeit von dem Ablauf der Schlauchpumpe bis zum Patienten reicht in der Regel nicht aus, damit die Pharmaka miteinander reagieren können. Die erfindungsgemäße Doppel-Blutpumpe ermöglicht es auch, relativ hochviskose Medikamente zu verarbeiten und mit anderen Medikamenten zu mischen.

Gemäß einer bevorzugten Ausführungsform der Erfindung sind die Taumelscheiben beider Schläuche mit entgegengesetztem Umdrehungssinn und gleichphasig zueinander angetrieben. Hierbei treten zwar die kurzzeitigen Förderunterbrechungen beider Pumpen zum gleichen Zeitpunkt auf, jedoch wird verhindert, daß die eine Pumpe vorübergehend in den Auslaß der anderen Pumpe hineinfördert. Die beiden Medien werden mit exakt gleichem Mischungsverhältnis gemischt, jedoch ist die Durchflußmenge pro Zeiteinheit nicht unbedingt konstant, sondern bei jedem Umlauf der Schlauchpumpe kurzzeitig unterbrochen, was bei den meisten Anwendungen akzeptiert werden kann.

Im folgenden werden unter Bezugnahme auf die Zeichnungen Ausführungsbeispiele der Erfindung näher erläutert.

Es zeigen:

Fig. 1    einen Längsschnitt durch eine Schlauchpumpe,
Fig. 2    einen Schnitt entlang der Linie II-II in Fig. 1,
Fig. 2a   eine Darstellung wie Fig. 2 bei einer abgeänderten Ausführungsform,

Fig. 3    eine teilweise Stirnansicht der Schlauchpumpe aus Richtung des Pfeiles III in Fig. 1,

Fig. 4    einen Längsschnitt durch den Arbeitsteil einer Doppel-Schlauchpumpe,

Fig. 5    eine Ansicht des Deckels der Doppel-Schlauchpumpe nach Fig. 4 in geöffnetem Zustand mit eingelegten Schläuchen und

Fig. 6    eine schematische Darstellung der Verwendung der Doppel-Schlauchpumpe der Fign. 4 und 5.

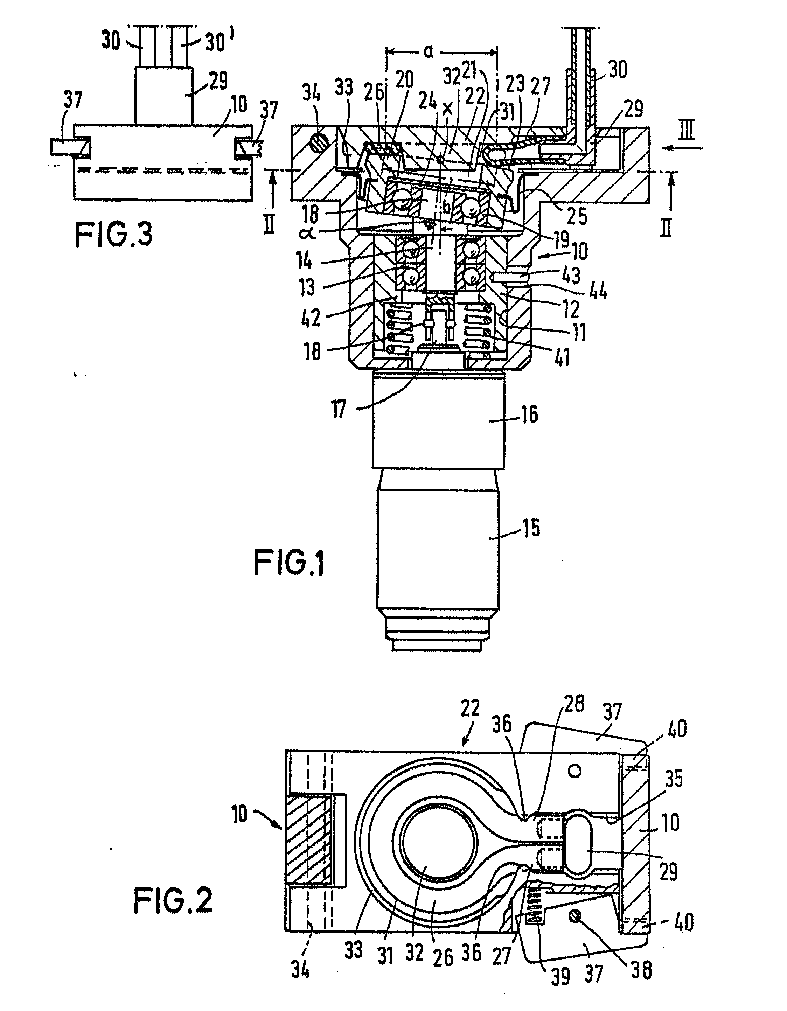

Die in den Fign. 1 bis 3 dargestellte Schlauchpumpe weist ein Gehäuse 10 mit einer zylindrischen Bohrung 11 auf, in der eine Hülse 12 längsverschiebbar gelagert ist. Im Innern der Hülse 12 ist mit Kugellagern 13 die Welle 14 gelagert, welche von einem Elektromotor 15 über ein Untersetzungsgetriebe 16 angetrieben ist. Die Antriebswelle 17 wird von der Ausgangswelle des Untersetzungsgetriebes 16 gebildet und sie ist über eine gabelförmige Kupplung 18 derart mit der Welle 14 gekuppelt, daß die Welle 14 kleine Axialverschiebungen relativ zu der Ausgangswelle 17 ausführen kann.

An ihrem abtriebsseitigen Ende weist die Welle 14 ein abgewinkeltes Wellenstück 18 auf, dessen Achse mit der Achse der Welle 14 einen spitzen Winkel α bildet. Auf diesem Wellenstück 18 ist über ein Kugellager 19 die Taumelscheibe 20 gelagert. Das Kugellager 19 und die Taumelscheibe 20 befinden sich in einer Kammer 21 im Innern des Gehäuses 10. Diese Kammer kann mit dem Deckel 22 verschlossen werden. Die Taumelscheibe 20 besteht bei dem vorliegenden Ausführungsbeispiel aus einem Ring, dessen äußere Stirnseite 23 entsprechend dem Winkel α abgeschrägt ist. Der Schnittpunkt der Achsen von Welle 14 und Wellenstück 18 liegt in der Ebene des mittleren Durchmessers b der Stirnseite 23, (d.h. des Durchmessers des Mittelkreises der konischen Fläche der Stirnseite 23).

Im Innern des Ringes sitzt das Kugellager 19, dessen äußere Stirnseite durch eine Dichtscheibe 24 abgedichtet ist, so daß keine Flüssigkeit durch den Axialkanal der Taumelscheibe 20 hindurchdringen kann. Am Umfang der Taumelscheibe 20 ist der innere Rand einer Membran 25 befestigt, deren äußerer Rand an der Seitenwand der Kammer 21 des Gehäuses 10 angebracht ist. Die Dichtungsscheibe 24 bildet daher zusammen mit der Membran eine flüssigkeitsdichte Abdichtung der Antriebseinrichtung der Taumelscheibe 20 gegen den den Schlauch 26 aufnehmenden Raum.

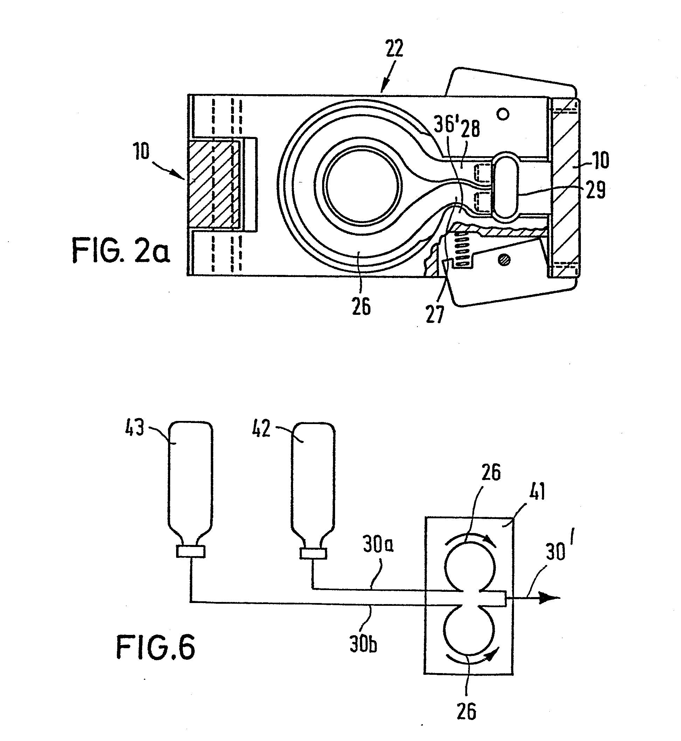

Der Schlauch 26 ist an der Unterseite des Deckels 22 befestigt. Wie aus Fig. 2 hervorgeht, ist der Schlauch 26 zu einem Ring gebogen, wobei der Einlaß 27 und der Auslaß 28 nebeneinander angeordnet sind und radial abstehen. Der Einlaß 27 und der Auslaß 28 sind mit einem Anschlußstück 29 verbunden, das im wesentlichen L-förmig ausgebildet ist und dessen Zulaufstutzen 30 und Ablaufstutzen 30' senkrecht aus dem Deckel 22 herausragen.

Der Schlauch 26 ist in eine ringförmige Nut 31 an der Unterseite des Deckels 22 eingelegt. Diese ringförmige Nut 31 wird innen durch einen axial vorstehenden Vorsprung 32 und außen durch einen umlaufenden Rand 33 von gleicher Tiefe begrenzt. Wie aus Fig. 1 ersichtlich ist, greift die ringförmige Taumelscheibe 20 in die ringförmige Nut 31 des Deckels 22 ein, wobei infolge der Schrägstellung der Taumelscheibe 20 die Eindringtiefe über den Umfang variiert. Zwischen der äußeren Stirnseite 23 der Taumelscheibe und dem Boden der ringförmigen Nut 31 wird der Schlauch 26 in axialer Richtung zusammengequetscht.

Wie aus Fig. 1 hervorgeht, ist der mittlere Umfang a der ringförmigen Stirnseite 23 der Taumelscheibe 20 gleich groß wie der mittlere Umfang b der ringförmigen Nut 31 bzw. der mittlere Umfang des Schlauchringes. Dadurch wird erreicht, daß die Stirnseite 23 eine reine Rollbewegung in der Nut 31 ausführt, ohne sich jedoch in dieser Nut zu drehen. Würden die mittleren Durchmesser a und b stärker voneinander abweichen, so würde die Taumelscheibe 20 dazu neigen, sich in dem Gehäuse langsam um die Achse des Wellenstückes 18 herum zu drehen. Dabei würde sie eine Schubwirkung auf den Schlauch ausüben, wodurch Reibungsverluste entstehen würden, die den Wirkungsgrad verschlechtern.

Der Deckel 22 ist an dem Gehäuse 10 mit einer Gelenkachse 34 befestigt, so daß er gemäß Fig. 2 aufgeklappt werden kann. Das Anschlußstück 29 befindet sich an dem der Gelenkachse 34 abgewandten Ende des Deckels 22. An diesem Ende ist ein Längsschlitz 35 vorgesehen. Der Schlauch 26, der von unten her in den Deckel 22 eingelegt ist, wird durch Laschen 36 festgehalten, die das einlaßseitige Ende und das auslaßseitige Ende des Schlauches 26 teilweise übergreifen. Zwischen diesen Laschen 36 befindet sich jedoch ein Spalt, der so breit ist, daß der Schlauch 26 unter relativ geringer Verformung aus dem Deckel 22 herausgenommen werden kann.

Die Ausführungsform der Fig. 2a entspricht weitgehend derjenigen der Fig. 2. Der einzige Unterschied besteht darin, daß anstelle der Laschen 36, die gemäß Fig. 2 den Schlauch 26 in dem Deckel festhalten, am Rand des Längsschlitzes 35 des Deckels 12 eine halbzylindrische Beule 36' vorgesehen ist, die das eine Schlauchende in der Nähe des Anschlußstückes 29 in Richtung auf das andere Schlauchende drückt. Auf diese Weise wird der Schlauch deformiert, wobei beide Schlauchenden leicht zusammengedrückt und in dem Deckel 22 durch Klemmung festgehalten werden.

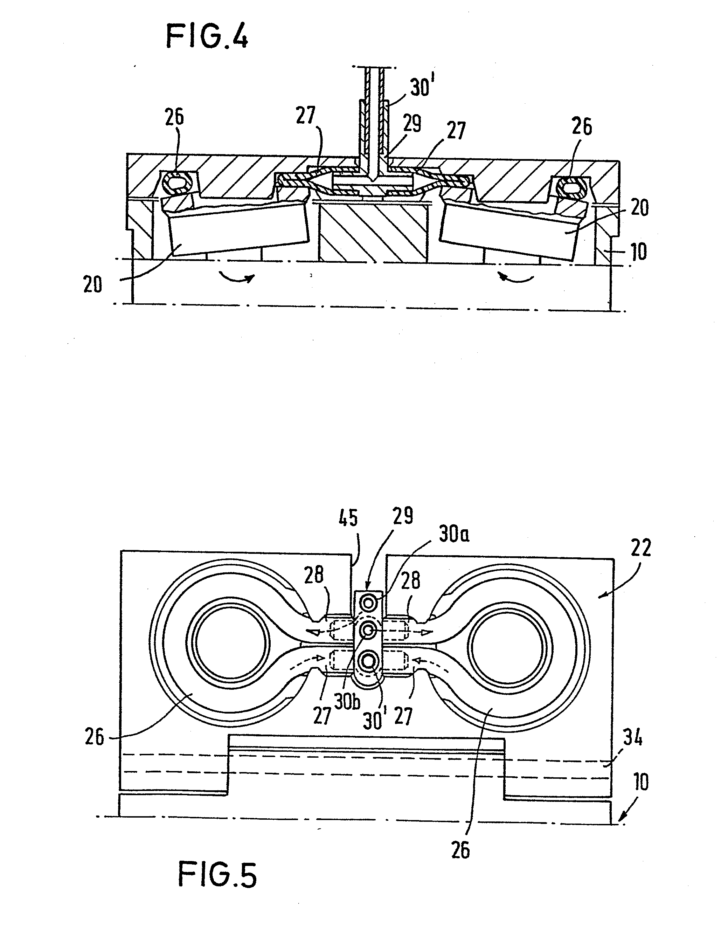

Zur Verriegelung des Deckels 22 in der Schließstellung am Gehäuse 10 dienen Klauen 37 an dem der Gelenkachse 34 abgewandten Ende des Deckels 22. Diese Klauen 37 sind jeweils mit einem Gelenkzapfen 38 an dem Deckel 22 gelagert und werden von einer Feder 39 in die Verriegelungsstellung gedrückt. Ihre äußeren Enden 40 greifen gemäß Fig. 3 in seitliche Ausnehmungen des Gehäuses 10 ein, um den Deckel 22 in der Schließstellung an dem Gehäuse 10 zu verriegeln. Werden die Enden 40 durch seitliches Eindrücken der Klauen 37 unter Zusammendrückung der Federn 39 auseinandergespreizt, dann wird der Deckel 22 am Gehäuse 10 entriegelt und er kann hochgeschwenkt werden.

Zur Ingebrauchnahme der Schlauchpumpe wird zunächst der Schlauch 26 zusammen mit dem Anschlußstück 29 in der in Fig. 2 dargestellten Weise in den Deckel 22 eingelegt. Dann wird der Deckel 22 geschlossen und an dem Gehäuse 10 verriegelt. Hierbei wird die Taumelscheibe 20 in ihrem am weitesten vorstehenden Bereich so fest gegen den Schlauch 26 gedrückt, daß sie diesen in der ringförmigen Nut 31 zusammenquetscht. Auf diese Weise entsteht entlang des Umfangs des von dem Schlauch 26 gebildeten Ringes eine einzige Quetschstelle. Diese ist in Umfangsrichtung so lang, daß an Ein- und Auslaß kein Kurzschluß entsteht; es werden kurzzeitig beide Schlauchenden 27 und 28 gemeinsam abgequetscht. Wird der Motor 15 eingeschaltet, dann dreht sich die Welle 14 und die Taumelscheibe 20 führt eine Taumelbewegung aus, ohne jedoch an der Drehung teilzunehmen. Infolge der Taumelbewegung wandert die Stelle, an der die maximale Zusammendrückung des Schlauches 26 stattfindet, entlang des von dem Schlauch 26 gebildeten Ringes mit konstanter Geschwindigkeit um. Auf diese Weise wird die im Schlauch 26 befindliche Flüssigkeit vom Einlaß 27 zum Auslaß 28 vorgetrieben.

Nach Beendigung der Behandlung werden die Klauen 37 gelöst und der Deckel 22 wird hochgeklappt. In diesem Zustand kann der Schlauch 26 zusammen mit dem Anschlußstück 29 entfernt und ersetzt werden.

Die Kugellager 13 und 24 zur Lagerung der Welle 14 bzw. der Taumelscheibe 20 dienen nicht nur als Radialkugellager, sondern sie sind auch imstande, axiale Kräfte zu übertragen, damit der Anpreßdruck der Feder 41 auf die Taumelscheibe 20 übertagen wird. Diese Feder 41 stützt sich an der rückwärtigen Stirnwand des Gehäuses 10 ab und drückt gegen eine Ringschulter 42 der Hülse 12, so daß die Hülse 12 zusammen mit der Welle 14 und den Kugellagern 13 und 19 sowie mit der Taumelscheibe 20 in Richtung auf den Schlauch 26 bzw. den Deckel 22 gedrückt wird. Um die Hülse 12 verdrehungssicher in dem Gehäuse 10 festzulegen, ist an der Hülse 12 ein radial abstehender Stift 43 vorgesehen, der in ein axiales Langloch 44 in der Wand des Gehäuses 10 hineinragt. Dieser Stift 43 dient auch als axialer Anschlag, damit die Taumelscheibe bei geöffnetem Deckel nicht heraus-

0149691

- 9 -

fällt und damit die Taumelscheibe bei geschlossenem Deckel, ohne Schlauch, nicht mit den Führungen 32 und 33 in Beziehung kommt und Beschädigungen hervorruft.

Da sich derjenige Teil der Taumelscheibe 20, der axial am weitesten vorsteht und die maximale Abquetschung des Schlauches 26 bewirkt, auf dem von dem Schlauch gebildeten Kreis umläuft, wird die Flüssigkeit von dem Einlaß 27 zum Auslaß 28 gefördert.

Bei dem Ausführungsbeispiel der Fign. 4 und 5 sind zwei Schlauchpumpen in einem gemeinsamen Gehäuse 10 nebeneinander angeordnet. Die Taumelscheiben 20 der beiden Schlauchpumpen sind gegensinnig zueinander angetrieben und ihre Einlässe 27 sind jeweils mit einem separaten Zulauf 30a bzw. 30b verbunden. Die Auslässe 28 sind untereinander verbunden und an den gemeinsamen Ablauf 30' angeschlossen. Das Anschlußstück 29 ist in einem Spalt 45 des Deckels 22 angeordnet, der an der der Gelenkachse 34 abgewandten Seite des Deckels 22 frei ausläuft.

Wie aus Fig. 4 hervorgeht, haben die beiden Taumelscheiben 20 keine gegenseitige Phasenverschiebung. Während die linke Taumelscheibe 20 die stärkste Abquetschung des zugehörigen Schlauches 26 gerade an der dem Anschlußstück 29 zugewandten Seite ausführt, bewirkt die rechte Taumelscheibe 20 die maximale Abquetschung zu derselben Zeit ebenfalls an der dem Anschlußstück 29 zugewandten Seite.

Der Deckel 22 nimmt bei dem Ausführungsbeispiel der Fign. 4 und 5 beide Schläuche 26, die in einer gemeinsamen Ebene liegen, gemeinsam auf. Die Schläuche 26 bilden zusammen mit dem zwischen ihnen angeordneten Anschlußstück 29 die Wegwerfeinheit.

In Fig. 6 ist die Verwendung der in den Fign. 4 und 5 dargestellten Doppel-Schlauchpumpe, die mit 41 bezeichnet ist, schematisch dargestellt. Aus zwei Behältern 42,43 werden der Doppel-Schlauchpumpe 41 unterschiedliche Medikamente zugeführt. Der Auslaß des Behälters 42 ist über einen Schlauch mit dem Zulauf 30a und der Behälter 43 ebenfalls über einen Schlauch mit dem Zulauf 30b der Schlauchpumpe 41 verbunden. Am Ausgang der Doppel-Schlauchpumpe 41 werden beide Flüssigkeiten miteinander vermischt und dem gemeinsamen Ablauf 30' zugeführt, der zu dem Patienten führt.

ANSPRÜCHE

1. Schlauchpumpe, insbesondere für medizinische Anwendungen, mit einem Gehäuse (10), in dem eine Antriebswelle (14) gelagert ist, einem von der Antriebswelle schräg abstehenden Wellenstück (18), einer auf dem Wellenstück (18) gelagerten und gegenüber dem Gehäuse (10) im wesentlichen verdrehungssicher festgehaltenen Taumelscheibe (20), einem an der Stirnseite der Taumelscheibe anliegenden ringförmigen Schlauch (26), dessen Enden einen Einlaß und einen Auslaß bilden, und mit einem an dem Gehäuse angebrachten, den Schlauch abstützenden Deckel (22),

   d a d u r c h   g e k e n n z e i c h n e t ,
   daß zwei Schläuche (26) seitlich nebeneinander in einer gemeinsamen Ebene angeordnet sind, daß jedem Schlauch eine eigene Taumelscheibe (20) zugeordnet ist, daß die Taumelscheiben (20) von einem gemeinsamen Antrieb mit gleichen Geschwindigkeiten angetrieben sind und daß beide Schläuche (26) separate Zuläufe und einen gemeinsamen Ablauf aufweisen.

2. Schlauchpumpe nach Anspruch 1, dadurch gekennzeichnet, daß beide Schläuche (26) in einem gemeinsamen Deckel (22) angeordnet sind.

3. Schlauchpumpe nach Anspruch 2, dadurch gekennzeichnet, daß die Taumelscheiben (20) beider Schläuche (26) gegensinnig und gleichphasig zueinander angetrieben sind.

4.  Schlauchpumpe nach Anspruch 1 oder 2, dadurch gekennzeichnet, daß die Taumelscheiben (20) beider Schläuche (26) mit gleichem Umdrehungssinn und phasemmäßig versetzt zueinander angetrieben sind.

5.  Schlauchpumpe nach Anspruch 1 oder 2, dadurch gekennzeichnet, daß die Taumelscheiben (20) beider Schläuche (26) mit entgegengesetztem Umdrehungssinn angetrieben sind.

0149691

1/3

FIG.3

FIG.1

FIG.2

FIG. 2a

22
36'28
10
10
29
26
27

FIG.6

43
42
26
41
30a
30'
30b
26

## FIG.4

## FIG.5

### Europäisches Patentamt

# EUROPÄISCHER RECHERCHENBERICHT

EP 84 10 0519

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR-A-2 520 639 (ISOJET) <br> * Figuren 1, 3; Ansprüche 1, 6 * | 1 | F 04 B 43/10 <br> F 04 B 43/12 <br> A 61 M 1/10 |
| A | FR-A-2 381 189 (NIKKISO EIKO CO.) <br> * Figuren 9, 10, 12; Ansprüche 1-3 * | 1 | |
| A | US-A-3 720 489 (D. RAPER) <br> * Figur 1; Zusammenfassung * | 1 | |
| A | GB-A-1 595 901 (IMPERIAL CHEMICAL INDUSTRIES) <br> * Figuren 1-2; Anspruch 1 * | 1,4 | |
| A | WO-A-8 204 291 (GRÄNDE et al.) <br> * Figuren 1-3; Zusammenfassung * | 1,4 | RECHERCHIERTE SACHGEBIETE (Int Cl 3) |
| A | EP-A-0 078 092 (BERELSON) <br> * Figuren 1-4 * | 1,4 | F 04 B 43/00 <br> F 04 C 5/00 <br> A 61 M 1/00 <br> A 61 M 5/00 |
| E | DE-A-3 227 051 (B. BRAUN MELSUNGEN AG) | 1 | |

---

Der vorliegende Recherchenbericht wurde für alle Patentansprüche erstellt.

| Recherchenort | Abschlußdatum der Recherche | Prüfer |
|---|---|---|
| BERLIN | 05-09-1984 | BARNY DE ROMANET P.M |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein- stimmendes Dokument